(19) Europäisches Patentamt
European Patent Office
Office européen des brevets

(11) EP 2 516 399 B1

# (12) FASCICULE DE BREVET EUROPEEN

(45) Date de publication et mention de la délivrance du brevet:
**18.04.2018 Bulletin 2018/16**

(21) Numéro de dépôt: **10808916.0**

(22) Date de dépôt: **22.12.2010**

(51) Int Cl.:
***C07D 213/74*** (2006.01) ***A61K 31/44*** (2006.01)
***A61K 8/49*** (2006.01) ***A61Q 19/00*** (2006.01)

(86) Numéro de dépôt international:
**PCT/FR2010/052872**

(87) Numéro de publication internationale:
**WO 2011/077044 (30.06.2011 Gazette 2011/26)**

## (54) DERIVES PHENOLIQUES, ET LEUR UTILISATION PHARMACEUTIQUE OU COSMETIQUE

PHENOLDERIVATE UND DEREN PHARMAZEUTISCHE SOWIE KOSMETISCHE VERWENDUNG

PHENOLIC DERIVATIVES AND PHARMACEUTICAL OR COSMETIC USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **23.12.2009 US 282152 P**
**23.12.2009 FR 0959476**

(43) Date de publication de la demande:
**31.10.2012 Bulletin 2012/44**

(73) Titulaire: **Galderma Research & Development**
**06410 Biot (FR)**

(72) Inventeurs:
- **POINSARD, Cédric**
**F-06130 Le Plan de Grasse (FR)**
- **COLLETTE, Pascal**
**F-06110 Le Cannet (FR)**
- **MAUVAIS, Pascale**
**F-06600 Antibes (FR)**
- **LINGET, Jean-Michel**
**F-67230 Benfeld (FR)**
- **RETHORE, Sandrine**
**F-06560 Valbonne (FR)**

(74) Mandataire: **Sekhri, Redha et al**
**Cabinet Becker & Associés**
**25, rue Louis le Grand**
**75002 Paris (FR)**

(56) Documents cités:
**WO-A1-2005/042464** **WO-A2-2006/010637**
**US-A- 4 578 390**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 516 399 B1

## Description

La présente invention a pour objet de nouveaux composés de formule générale :

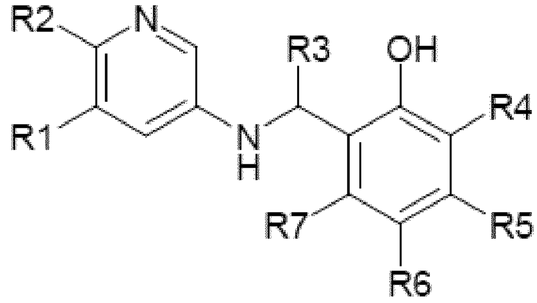

(I)

Et leur utilisation cosmétique ou pharmaceutique.

La présente invention se propose de fournir de nouveaux dérivés phénoliques, qui soient de puissants modulateurs du récepteur aux androgènes.

Parmi les documents de l'art antérieur décrivant des molécules modulant l'activité du récepteur aux androgènes, on peut, par exemple, citer les phenylimidazolines décrites dans la demande de brevet EP580459, ou la demande WO200542464.

WO 2006/010637 décrit des pyridinylamines inhibiteurs de kinase qui peuvent être utilisés dans le traitement de diverses maladies, notamment l'alopécie et l'acné.

US 4,578,390 décrit des dérivés hydroxybenzylamino en tant qu'agents anti-inflammatoires, et notamment des dérivés 1-amino-pyridyles dont la fonction amino est substituée par un groupe 2-hydroxybenzyle.

La présente invention a pour objet les revendications 1 à 9.

La présente divulgation décrit des dérivés phénoliques qui répondent à la formule générale (I) suivante :

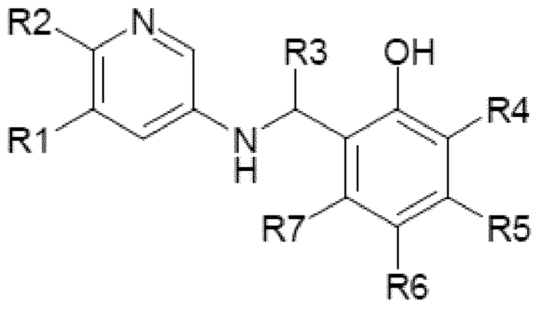

(I)

dans laquelle :

- $R_1$ représente un groupe $C_{1-6}$ alkyle, $C_{3-7}$ cycloalkyle, $C_{1-6}$ alkyloxy, $-S(O)_m-C_{1-6}$ alkyle, $C_{1-6}$ fluoroalkyle, $C_{1-6}$ fluoroalkyloxy, $-(CH_2)_n-C_{3-9}$ cycloalkyle, $-(CH_2)_n-C_{3-9}$ cycloalkyle, $C_{2-6}$ alkyle-OH, $-(CH_2)_n-C_{1-6}$ alkyloxy, $-(CH_2)_n-C_{1-6}$ fluoroalkyle, $-(CH_2)_p-O-C_{1-6}$ fluoroalkyle, $COR_a$, CN, $NO_2$, $NR_8R_9$, un halogène, ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_b$ identiques ou différents
- $R_2$ représente un groupe $C_{1-6}$ alkyle, $C_{3-7}$ cycloalkyle, $C_{1-6}$ alkyloxy, $-S(O)_v-C_{1-6}$ alkyle, $C_{1-6}$ fluoroalkyle, $C_{1-6}$ fluoroalkyloxy, $-(CH_2)_q-C_{3-9}$ cycloalkyle, $-(CH_2)_q-C_{3-9}$ cycloalkyle, $C_{2-6}$ alkyle-OH, $-(CH_2)_q-C_{1-6}$ alkyloxy, $-(CH_2)_q-C_{1-6}$ fluoroalkyle, $-(CH_2)_r-O-C_{1-6}$ fluoroalkyle, $COR_d$, CN, $NO_2$, $NR_8'R_9'$, un atome d'hydrogène, un halogène, ou un groupe phenyle ou heteroaryle contenant soit a) de 1 à 4 atomes d'azote soit b) un atome d'oxygène ou de soufre et 1 ou 2 atomes d'azote. Ces groupes phenyles et heteroaryles peuvent être éventuellement substitués par un à trois groupes $R_e$ identiques ou différents
- $R_3$ représente un atome d'hydrogène ou un groupe $C_{1-9}$ alkyle, $C_{3-9}$ cycloalkyle, $C_{1-6}$ fluoroalkyle, $-(CH_2)_t-C_{3-9}$ cycloalkyle, $-C_{2-6}$ alkyle-OH, $-(CH_2)_u-C_{1-6}$ alkyloxy, $-(CH_2)_t-C_{3-7}$ cycloalkyle, $-(CH_2)_t-C_{1-6}$ fluoroalkyle, ou $-(CH_2)_u-O-$

$C_{1-6}$ fluoroalkyle

- $R_4$, $R_5$, $R_6$, $R_7$ sont identiques ou différents et représentent soit un atome d'hydrogène soit un groupe $C_{1-6}$ alkyle, $C_{3-7}$ cycloalkyle, $C_{1-6}$ alkyloxy, $-S(O)_l-C_{1-6}$ alkyle, $C_{1-6}$ fluoroalkyle, $C_{1-6}$ fluoroalkyloxy, $-(CH_2)_i-C_{3-9}$ cycloalkyle, $-(CH_2)_i-C_{3-9}$ cycloalkyle, $-C_{1-6}$ alkyle -OH, $-(CH_2)_i-C_{1-6}$ alkyloxy, $-(CH_2)_i-C_{1-6}$ fluoroalkyle, $-(CH_2)_j-O-C_{1-6}$ fluoroalkyle, $COR_a$, CN, $NR_{10}R_{11}$, ou un halogène ou un groupe phenyle ou heteroaryle contenant soit a) de 1 à 4 atomes d'azote soit b) un atome d'oxygène ou de soufre et 1 ou 2 atomes d'azote. Ces groupes phenyle et heteroaryle peuvent être éventuellement substitués par un à trois groupes $R_c$ identiques ou différents
- $R_a$, $R_d$ et $R_f$ sont identiques ou différents et représentent un groupe $C_{1-6}$ alkyle, $C_{1-6}$ alkyloxy ou $NR_{12}R_{13}$
- $R_b$, $R_c$ et $R_e$ sont identiques ou différents et représentent un halogène, un groupe $C_{1-6}$ alkyle, $C_{3-7}$ cycloalkyle, $C_{1-6}$ alkyloxy, $-S(O)_k-C_{1-6}$ alkyle, $C_{1-6}$ fluoroalkyle, $C_{1-6}$ fluoroalkyloxy, $-(CH_2)_g-C_{3-7}$ cycloalkyle, OH, $-(CH_2)_g-C_{3-7}$ cycloalkyle, $C_{1-6}$ alkyle-OH, $-(CH_2)_g-C_{1-6}$ alkyloxy, $-(CH_2)_g-C_{1-6}$ fluoroalkyle, $-(CH_2)_w-O-C_{1-6}$ fluoroalkyle, $COR_f$, CN, ou $NR_{14}R_{15}$
- $R_8$, $R_8$', $R_9$, $R_9$', $R_{10}$, $R_{11}$, $R_{12}$, $R_{13}$, $R_{14}$ et $R_{15}$ sont identiques ou différents et représentent un atome d'hydrogène, un groupe $C_{1-6}$ alkyle, $C_{3-7}$ cycloalkyle, $-(CH_2)_h-C_{3-7}$ cycloalkyle ou $-(CH_2)_h-C_{1-6}$ fluoroalkyle.

Eventuellement les groupes $R_8$ et $R_9$ peuvent former avec l'atome d'azote qui les porte un hétérocycle tel que : azetidine, pyrolidine, pipéridine, azepane, morpholine ou pipérazine. Eventuellement les groupes $R_8$' et $R_9$' peuvent former avec l'atome d'azote qui les porte un hétérocycle tel que : azetidine, pyrolidine, pipéridine, azepane, morpholine ou pipérazine. Eventuellement les groupes $R_{10}$ et $R_{11}$ peuvent former avec l'atome d'azote qui les porte un hétérocycle tel que : azetidine, pyrolidine, pipéridine, azepane, morpholine ou pipérazine. Eventuellement les groupes $R_{12}$ et $R_{13}$ peuvent former avec l'atome d'azote qui les porte un hétérocycle tel que : azetidine, pyrolidine, pipéridine, azepane, morpholine ou pipérazine. Eventuellement les groupes R14 et R15 peuvent former avec l'atome d'azote qui les porte un hétérocycle tel que : azetidine, pyrolidine, pipéridine, azepane, morpholine ou pipérazine

- g, h, i, n, q et t sont différents ou identiques et sont égales à 1, 2 ou 3
- k, l, m et v sont différents ou identiques et sont égales à 0, 1 ou 2
- j, p, r, u et w sont différents ou identiques et sont égales à 2, 3 ou 4

ainsi que leurs sels pharmaceutiquement acceptables, solvates ou hydrates et leurs conformères ou rotamères.

Les composés de formule (I) peuvent comporter un ou plusieurs atomes de carbone asymétriques. Ils peuvent donc exister sous forme de mélange d'énantiomères ou de diastéréoisomères. Ces énantiomères, diastéréoisomères ainsi que leurs mélanges y compris les mélanges racémiques font partie de l'invention.

Les composés de formule (I) peuvent exister à l'état de base ou de sels d'addition à des acides. De tels sels d'addition font partie de l'invention. Ces sels sont avantageusement préparés avec des acides pharmaceutiquement acceptables mais les sels d'autres acides utiles, par exemple pour la purification ou l'isolement des composés de formule (I) font également partie de l'invention. Ces acides peuvent être par exemple l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate. Pour une revue des sels physiologiquement acceptables voir Handbook of Pharmaceutical Salts : Properties, Selection and Use de Stahl et Wermuth (Wiley-VCH, 2002).

Les solvates ou hydrates pourront être obtenus directement à l'issue du procédé de synthèse, le composé (I) étant isolé sous la forme d'un hydrate, par exemple un mono ou hémi-hydrate ou d'un solvate du solvant de réaction ou de purification.

Dans le cadre de la présente divulgation on entend par :

- $C_{b-}$c où b et c peuvent prendre des valeurs de 1 à 9, une chaine carbonnée de b à c atomes de carbone, par exemple $C_{1-}$6 une chaine carbonnée pouvant avoir 1 à 6 atomes de carbon - alkyle, un groupe aliphatique saturé linéaire ou ramifié, par exemple un groupe $C_{1-}$6 alkyle représente une chaine carbonnée de 1 à 6 atomes de carbone, linéaire ou ramifiée, par exemple un méthyle, éthyle, propyle, isopropyle, butyle, isobutyle, terbutyle, pentyle, hexyle - cycloalkyle, une chaine carbonnée saturée cyclique éventuellement ramifiée, comportant de 3 à 7 atomes de carbone. A titre d'exemple un groupe $C_{3-}$7 cycloalkyle représente une chaine carbonnée de 3 à 7 atomes de carbone par exemple un cyclopropyle, cyclobutyle, cyclopentyle, cyclohexyle et cycloheptyle
- hétérocycle, une chaine hydrocarbonée cyclique ou bicylique, saturée ou insaturée, comprenant un ou plusieurs hétéroatomes choisis parmi O, S et N
- heteroaryle, un hétérocycle aromatique, par exemple un groupe pyridinyle, pyrazinyle, pyrimidinyle, pyridazinyle, triazinyle, pyrazolyle, isooxazolyle, oxadiazolyle, thiazolyle, thiadiazolyle, triazolyle, ou imidazolyle
- halogène, un atome de fluore, de chlore ou de brome

- alkyloxy, un groupe -O-alkyle
- alkylthio, un groupe -S-alkyle
- fluoroalkyle, un groupe alkyle dont un ou plusieurs atomes d'hydrogène ont été remplacés par un atome de fluor
- fluoroalkyloxy, un groupe alkyloxy dont un ou plusieurs atomes d'hydrogène ont été remplacés par un atome de fluor

Est préféré le groupe (A) des composés de formule (I) ci-dessus définis, dans lesquels le groupe $R_1$ représente un halogène, un groupe méthyle, éthyle, isopropyle, trifluorométhyle, nitrile, nitro, methoxy, ethoxy, isopropoxy, thiométhyle, thioéthyle, thio isopropyle et plus particulièrement tels que $R_1$ représente un halogène, un groupe methoxy, ethoxy, thiométhyle, thioéthyle ou trifluorométhyle.
Le groupe (B) des composés de formule (I) dont le substituant $R_1$ est définis ci-dessus ou dans le groupe préféré (A) et tels que le groupe R2 est un atome d'hydrogène.
Est particulièrement préféré le groupe (C) des composés de formule (I) dont les substituants $R_1$ et $R_2$ sont définis ci-dessus ou dans les groupes préférés (A) ou (B) et tels que le groupe $R_3$ est un atome d'hydrogène ou un groupe $C_{1-6}$ alkyle.
Les composés ci-dessous, ainsi que leurs sels pharmaceutiquement acceptables, solvates et hydrates et leurs conformères ou rotamères sont particulièrement préférés :

2-[(5-Bromo-pyridin-3-ylamino)-methyl]-phenol
2-[(5-Bromo-pyridin-3-ylamino)-methyl]-3-fluoro-phenol
2-[(5-Bromo-pyridin-3-ylamino)-methyl]-4-fluoro-phenol
2-[(5-Methyl-pyridin-3-ylamino)-methyl]-phenol
2-[(5-Bromo-pyridin-3-ylamino)-methyl]-5-fluoro-phenol
2-[(5-Bromo-pyridin-3-ylamino)-methyl]-3,5-dichloro-phenol
2-[(5-Bromo-pyridin-3-ylamino)-methyl]-4-chloro-phenol
2-[(5-Bromo-pyridin-3-ylamino)-methyl]-4,6-difluoro-phenol
2-[1-(5-Bromo-pyridin-3-ylamino)-propyl]-phenol
2-[1-(5-Bromo-pyridin-3-ylamino)-ethyl]-4-fluoro-phenol
2-[(5-Methoxy-pyridin-3-ylamino)-methyl]-phenol
2-[1-(5-Bromo-pyridin-3-ylamino)-ethyl]-phenol
2-[(5-Bromo-pyridin-3-ylamino)-methyl]-3,4-difluoro-phenol
5-(2-Hydroxy-benzylamino)-nicotinonitrile
2-[(5-Chloro-pyridin-3-ylamino)-methyl]-phenol
2-[1-(5-Bromo-pyridin-3-ylamino)-butyl]-phenol
2-[1-(5-Bromo-pyridin-3-ylamino)-pentyl]-phenol
2-[(5-Bromo-6-methyl-pyridin-3-ylamino)-methyl]-phenol
2-[(5-Bromo-6-methoxy-pyridin-3-ylamino)-methyl]-phenol
5-(2-Hydroxy-benzylamino)-3-methyl-pyridine-2-carbonitrile
2-[(6-Methoxy-5-methyl-pyridin-3-ylamino)-methyl]-phenol
2-[(6-Chloro-5-methyl-pyridin-3-ylamino)-methyl]-phenol
2-[(6-Bromo-5-methyl-pyridin-3-ylamino)-methyl]-phenol
2-[(5,6-Dimethyl-pyridin-3-ylamino)-methyl]-phenol
2-[(5-Methyl-6-trifluoromethyl-pyridin-3-ylamino)-methyl]-phenol

La présente divulgation décrit également un procédé de préparation des composés de formule générale (I).
Conformément à l'invention on peut préparer les composés de formule (I) selon le procédé général décrit dans le **Schéma 1** ci après.

## Schéma 1

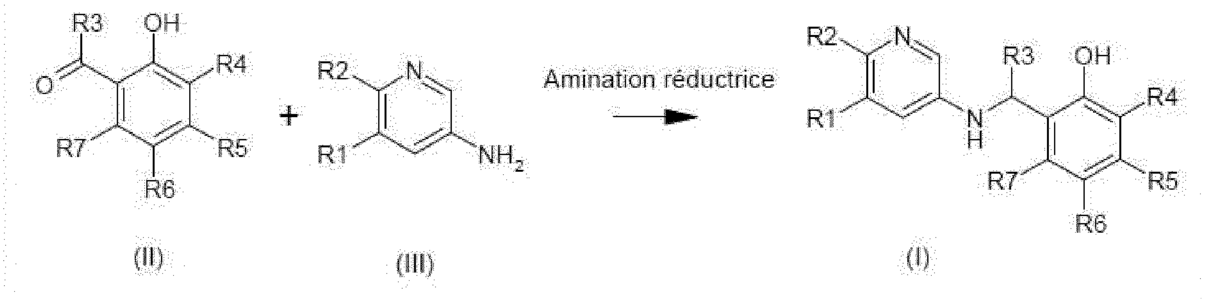


Les composés phénoliques de formule (I) dans laquelle $R_1$, $R_2$, $R_3$, $R_4$, $R_5$, $R_6$ et $R_7$ sont tels que définis ci-dessus, peuvent être préparés par réaction d'amination réductrice entre un aldéhyde ou une cétone benzylique (II) et une amine (III) en présence d'un agent réducteur, tel que par exemple et de façon non limitante, le triacétoxyborohydrure de sodium dans par exemple un solvent tel que le dichlorométhane, selon le **Schéma 1** et par analogie aux réactions décrites, par exemple, dans Org. Pro. R. & D. (2006) 971 - 1031.

Les groupes fonctionnels éventuellement présents dans les intermédiaires réactionnels utilisés dans le procédé peuvent être protégés, soit de façon permanente, soit de façon temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, des alcools ou des acides carboxyliques on entend les groupes protecteurs tels que ceux décrits dans « Protective Groups in Organic Chemistry », ed McOmie J. W. F., Plenum Press, 1973, dans « Protective Groups in Organic Synthesis », 2nde édition, Greene T.W. et Wuts P.G.M., ed John Wiley et Sons, 1991 et dans « Protecting Groups », Kocienski P.J., 1994, Georg Thieme Verlag.

Les produits objets de la présente invention sont doués de propriétés pharmacologiques intéressantes ; on a constaté notamment qu'ils modulaient l'activité du récepteur aux androgènes.

Des tests donnés dans la partie expérimentale illustrent cette activité modulatrice du récepteur aux androgènes. Les produits objets de la présente invention présentent des activités d'antagonisme ou d'agonisme partiel ou total. Du fait de cette activité, les produits de l'invention peuvent être utilisés comme médicaments chez l'homme ou l'animal. Ces propriétés rendent les produits de formule générale (I) de la présente invention utilisables comme médicaments pour le traitement des cancers hormonaux dépendant tels que le cancer de la prostate ou le cancer du sein, ainsi que pour lutter contre l'hyperplasie bénigne de la prostate, la puberté précoce, la virilisation, le syndrome des ovaires polykystiques, syndrome de Stein-Leventhal, la perte de libido, l'endométriose. Les composés présentant une activité d'agonisme partiel ou total peuvent en particulier être utilisés pour traiter les afflictions telles que la perte de masse musculaire (sarcopénie), l'atrophie musculaire, l'impuissance et la stérilité masculine, la différentiation masculine anormale (hermaphrodisme), l'hypogonadisme, l'ostéoporose.

Les produits de formule générale (I) de l'invention trouvent également leur utilisation cosmétique pour l'hygiène corporelle ou capillaire.

Les produits de formule générale (I) de l'invention trouvent également leur utilisation dans le traitement de l'hirsutisme, de l'acné, de la séborrhée, la peau grasse de l'alopécie androgénique, de l'hyperpilosité ou hirsutisme, et ils peuvent être utilisés pour la fabrication d'un médicament pour prévenir et/ou traiter l'hirsutisme, l'alopécie androgénique, de l'hyperpilosité, la dermatite atopique, les désordres de la glande sébacée tels que l'hyperséborrhée, l'acné, la peau grasse ou la dermite séborrhéique. Les produits de formule (I) peuvent donc être utilisés en dermatologie : ils peuvent être utilisés seuls ou en association. Ils peuvent être associés notamment avec un produit antibiotique tels que les dérivés de l'acide azélaique, fusidique, l'érythromycine ou avec un dérivé des rétinoïdes tel que la tretinoïne pour le traitement de l'acné, ou avec un inhibiteur de la 5 a-réductase tel que le (5alpha,17beta)-N-1,1- diméthyléthyl 3-oxo 4-aza-androst-1-ène 17-carboxamide (ou Finastéride Merck, l3ème édition) ou l'acide azélaïque ou un agent bloquant des récepteurs androgènes pour le traitement de l'acné, de l'alopécie ou de l'hirsutisme, ou avec un produit stimulant la croissance des cheveux tel que le Minoxidil pour le traitement de l'alopécie.

La présente invention a également pour objet à titre de médicament les composés de formule (I) tels que décrits ci-dessus, ainsi que leurs sels pharmaceutiquement acceptables, solvates pharmaceutiquement acceptables et/ou hydrates.

## MODES OPERATOIRES

### Exemple 1 : 2-[(5-Bromo-pyridin-3-ylamino)-methyl]-phenol

295 mg (1.4 mmol, 1.4 eq) de triacetoxyborohydrure de sodium sont ajoutés à une solution de 173 mg (1 mmol, 1 eq) de 5-bromo-pyridin-3-ylamine (**produit de départ 1**) et 122 mg (1 mmol, 1 eq) de 2-hydroxy-benzaldehyde (**produit de départ 2**) dans 20 ml de dichlorométhane. La solution est agitée à température ambiante pendant 24h. Le milieu est versé dans de l'eau et agité pendant 2 h. La phase aqueuse est extraite avec du dichlorométhane. Les phases organiques sont rassemblées et lavées à l'eau puis séchées sur sulfate de sodium. Le résidu est chromatographié sur gel de silice (heptane / acétate d'éthyle 80/20). 2-[(5-bromo-pyridin-3-ylamino)-methyl]-phenol est obtenu sous la forme d'un solide beige.
Point de fusion = 184°C
RMN 1H (DMSO) 4.19 (d, 2H, J= 5.8 Hz); 6.62- 6.65 (m, 1H); 6.74 (t, 1H, J= 7.4 Hz); 6.82-6.84 (d, 1H, J= 9.0 Hz); 7.05-7.09 (m, 2H); 7.15- 7.17 (d, 1H, J= 8.8 Hz); 7.77 (s, 1H); 7.95 (s, 1H); 9.62 (s, 1H).

### Exemple 2 à 15

Les exemples 2 à 15 sont décrits dans le tableau 1 ci-dessous. Les composés sont synthétisés suivant les modes opératoires ci-dessus, en remplaçant les **produits de départ 1 et 2** évoqués dans l'exemple 1 par les produits mentionnés dans le tableau 1.

**Tableau 1**

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet , J= constante de couplage en Hz) |
|---|---|---|---|---|---|
| 2 | 2-[(5-Bromo-pyridin-3-ylamino)-methyl]-3-fluoro-phenol | 5-Bromo-pyridin-3-ylamine | 2-Fluoro-6-hydroxy-benzaldehyde | 193-195 | (DMSO) 4.17-4.19 (d, 2H, J= 4.8 Hz); 6.43-6.46 (m, 1H); 6.64 (t, 1H, J= 8.6 Hz); 6.7 (d, 1H, J= 8.2 Hz); 7.10-7.16 (m, 1H); |
| 3 | 2-[(5-Bromo-pyridin-3-ylamino)-methyl]-4-fluoro-phenol | 5-Bromo-pyridin-3-ylamine | 5-Fluoro-2-hydroxy-benzaldehyde | 192-194 | 7.24 (s, 1H); 7.77 (s, 1H); 8.02 (s, 1H); 10.24 (s, 1H) (DMSO) 4.2 (d, 2H, J= 6.0 Hz); 6.67- 6.7 (m, 1H); 6.80-6.83 (m, 1H); 6.87- 6.92 (m, 1H); 6.95-6.98 (m, 1H); 7.07- 7.11 (m, 1H); 7.80 (s, 1H); 7.95 (s, 1H); 9.67 (s, 1H) |
| 4 | 5-(2-Hydroxybenzylamino)-nicotinonitrile | 5-Amino-nicotinonitrile | 2-Hydroxy-benzaldehyde | Non déterminé | (DMSO) 4.22 (d, 2H, J= 4.8 Hz); 6.82 (t, 1H, J= 7.4 Hz); 6.83-6.85 (m, 2H); 7.08 (t, 1H, J= 7.7); 7.17 (d, 1H, J= 7.4 Hz); 7.23 (s, 1H); 8.07 (s, 1H); 8.24 (s, 1H); 9.68 (s, 1H) |

(suite)

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet , J= constante de couplage en Hz) |
|---|---|---|---|---|---|
| 5 | 2-[(5-Bromo-pyridin-3-ylamino)-methyl]-5-fluoro- | 5-Bromo-pyridin-3-ylamine | 4-Fluoro-2-hydroxy-benzaldehyde | 172 | (CD3OD) 4.27 (s, 2H); 6.50-6.58 (m, 2H); 7.16-7.25 (m, 2H); 7.77 (s, |
| 6 | 2-[(5-Bromo-pyridin-3-ylamino)-methyl]-3,5-dichloro-phenol | 5-Bromo-pyridin-3-ylamine | 2,4-Dichloro-6-hydroxy-benzaldehyde | 182 | 1H); 7.88 (s, 1H) (DMSO) 4.22 (d, 2H, J= 4.8 Hz); 6.36-6.38 (m, 1H); 6.88 (s, 1H); 7.06 (s, 1H); 7.26 (s, 1H); 7.79 (s, 1H); 8.03 (s, 1H); 10.83 (s, 1H) |
| 7 | 2-[(5-Bromo-pyridin-3-ylamino)-methyl]-4-chloro-phenol | 5-Bromo-pyridin-3-ylamine | 5-Chloro-2-hydroxy-benzaldehyde | 226-228 | (CD3OD) 4.29 (s, 2H); 6.79 (d, 1H, J=8.6Hz); 7.07 (m, 1H); 7.16-7.24 (m, 2H); 7.80 (s, 1H); 7.88 (s, 1H) |
| 8 | 2-[(5-Bromo-pyridin-3-ylamino)-methyl]-4,6-difluoro-phenol | 5-Bromo-pyridin-3-ylamine | 3,5-Difluoro-2-hydroxy-benzaldehyde | Non déterminé | (DMSO) 4.27 (s, 2H); 6.73 (m, 1H); 6.86 (d, 1H, J= 9.2 Hz); 7.06-7.12 (m, 2H); 7.81 (s, 1H); 7.94 (s, 1H); 9.81 (s, 1H) |
| 9 | 2-[1-(5-Bromo-pyridin-3-ylamino)-propyl]- | 5-Bromo-pyridin-3-ylamine | 1-(2-Hydroxy-phenyl)-propan-1-one | 215-217 | (CH3OD) 0.97 (t, 3H) ; 1.84 (m, 2H) ; 4.6 (t, 1H) ; 6.75 (m, 2H) ; 7-7.2 (m, 3H) ; 7.67 |
| 10 | 2-[1-(5-Bromo-pyridin-3-ylamino)-ethyl]-4-fluoro-phenol | 5-Bromo-pyridin-3-ylamine | 1-(5-Fluoro-2-hydroxy-phenyl)-ethanone | 175-177 | (s, 1H) ; 7.81 (s, 1H) (DMSO) 1.38 (d, 3H, J= 6.7 Hz); 4.67-4.73 (m, 1H); 6.74-6.96 (m, 5H); 7.76 (s, 1H); 7.83 (s, 1H); 9.72 (s, 1H) |
| 11 | 2-[(5-Methoxy -pyridin-3-ylamino)-methyl]-phenol | 5-Methoxy-pyridin-3-ylamine | 2-Hydroxy-benzaldehyde | 188-190 | (DMSO) 3.70 (s, 3H); 4.18 (d, 2H, J= 5.4 Hz); 6.30-6.33 (m, 1H); 6.46 (d, 1H, J= 4.6 Hz); 6.73 (t, 1H, J= 7.4 Hz); 6.82 (d, 1H, J= 7.4 Hz); 7.03-7.07 (m, 1H); 7.17 (d, 1H, J= 7.4 Hz); 7.46 (s, 1H); 7.60 (s, 1H); 9.59 (s, 1H) |

(suite)

| Exemple # | Nom IUPAC | Produit de départ 1 | Produit de départ 2 | Point de fusion (°C) | RMN 1H-400Mhz (s= singulet, d= doublet, t=triplet, m=multiplet, q=quadruplet , J= constante de couplage en Hz) |
|---|---|---|---|---|---|
| 12 | 2-[1-(5-Bromo-pyridin-3-ylamino)-ethyl]-phenol | 5-Bromo-pyridin-3-ylamine | 2'-hydroxyacetophenone | 178-180 | (DMSO) 1.38 (d, 3H, J= 6.7 Hz); 4.68-4.75 (m, 1H); 6.70-6.76 (m, 2H); 6.81 (d, 1H, J= 8.0 Hz); 6.87 (d, 1H, J= 4.2 Hz); 7.02 (t, 1H, J= 7.9 Hz); 7.15 (d, 1H, J= 9.0 Hz); 7.72 (s, 1H); 7.83 (s, 1H); 9.63 (s, 1H) |
| 13 | 2-[(5-Bromo-pyridin-3-ylamino)-methyl]-3,4-difluoro-phenol | 5-Bromo-pyridin-3-ylamine | 5-6-Difluoro-2-hydroxy-benzaldehyde | 194-196 | (CD3OD) 4.37 (s, 2H); 6.57-6.61 (m, 1H); 6.97-7.03 (m, 1H); 7.37 (s, 1H); 7.77 (s, 1H); 7.96 (s, 1H) |
| 14 | 2-[(5-Methyl-pyridin-3-ylamino)-methyl]-phenol | 5-Methyl-pyridin-3-ylamine | 2-Hydroxy-benzaldehyde | 183-185 | (DMSO) 2,13 (s, 3H); 4.18 (d, 2H, *J*=5.8Hz); 6.15-6.18 (m, 1H); 6.70-6.75 (m, 2H); 6.82 (d, 1H, *J*=8Hz); 7.05 (t, 1H, *J*=7.7Hz); 7.16 (d, 1H, *J*=7.4Hz); 7.57 (s, 1H); 7.77 (s, 1H); 9.55 (s, 1H) |
| 15 | 2-[(5-Chloro-pyridin-3-methyl]-phenol | 5-Chloro-pyridin-3-ylamine | 2-Hydroxy-benzaldehyde | 231-213 | (DMSO) 4.36 (d, 2H, *J*=5.8Hz); 6.55 (d, 1H, *J*=5.5Hz); 6.60 (s, 1H); 6.74 (t, 1H, *J*=7.4Hz); 7.15 (d, 1H, *J*=7.3Hz); 7.22-7.24 (m, 1H); 7.92 (d, 1H, *J*=5.5Hz); 9.82 (s, 1H) |

Tous les spectres de RMN (résonance magnétique nucléaire) sont en accord avec les structures proposées. Les déplacements chimiques sont exprimés en partie par million. La référence interne est le tetraméthylsilane. Les abréviations suivantes sont utilisées : CDCl3 = chloroforme deutérié, DMSO = diméthylsulphoxide deutérié, CD3OD = méthanol deutérié.

## Exemple 16 : Tests biologiques

Les composés selon l'invention présentent des propriétés inhibitrices des récepteurs de type AR. Cette activité inhibitrice des récepteurs AR est mesurée dans un test de transactivation par les constantes de dissociation KdR (repos), KdA (actif) et Kdapp (apparent) d'après la méthode exposée dans J. Molecular Biology (1965), 12(1), 88-118, Monod J. *et al.*

Par inhibiteur des récepteurs de type ARs, on entend selon l'invention tout composé qui présente une constante de dissociation Kdapp inférieure ou égale à 1 $\mu$M, et un rapport KdR / KdA $\leq$ 10, dans un test de transactivation.

Les composés préférés de la présente invention présentent une constante de dissociation inférieure ou égale

à 500 nM, et avantageusement inférieure ou égale à 100 nM.

Le test de transactivation est réalisé dans la lignée cellulaire PALM (PC3 Androgene receptor Luciferase MMTV) qui est un transfectant stable contenant les plasmides PMMTV-neo-Luc (gène reporter) et pSG5puro-AR.

Dans cette étude, l'affinité de chaque produit pour les 2 états des récepteurs (KdR et KdA) est déterminée ainsi qu'un Kd apparent (KdApp). Cette constante est une résultante des 2 Kd mais dépend également de l'équilibre initial du récepteur entre l'état actif et l'état repos ($L_0$) et de son taux d'expression. Sa détermination se fait par la formule suivante :

$$1/KdApp=(L0/(1+L0))x(1/KdR) +(1/(1+L0))x(1/KdA)$$

Pour déterminer ces constantes, des « courbes croisées » du produit à tester contre un agoniste de référence, le methyltrienolone, sont réalisées en plaque 96 puits. Le produit à tester est utilisé à 10 concentrations et l'agoniste de référence à 7 concentrations.

A titre illustratif, un Kdapp de 6 nM est obtenu pour le composé (1), un Kdapp de 5nM est obtenu pour le composé (2), un Kdapp de 200 nM est obtenu pour le composé (4), un Kdapp de 60 nM est obtenu pour le composé (9), un Kdapp de 15 nM est obtenu pour le composé (14).

## Revendications

**1.** Composés de formule (1) :

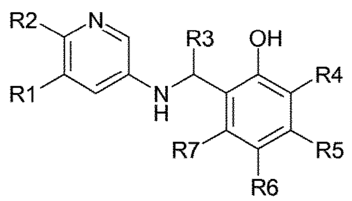

(I)

dans laquelle :

- R1 représente un halogène, un groupe méthyle, éthyle, isopropyle, trifluorométhyle, nitrile, nitro, methoxy, ethoxy, iso-propoxy, thiométhyle, thioéthyle ou thio isopropyle ;
- $R_2$ représente un atome d'hydrogène ;
- R3 représente un atome d'hydrogène ou un groupe $C_{1-6}$ alkyle ;
- $R_4$, $R_5$, $R_6$, $R_7$ sont identiques ou différents et représentent soit un atome d'hydrogène soit un groupe $C_{1-6}$ alkyle, $C_{3-7}$ cycloalkyle, $C_{1-6}$ alkyloxy, $-S(O)_1-C_{1-6}$ alkyle, $C_{1-6}$ fluoroalkyle, $C_{1-6}$ fluoroalkyloxy, $-(CH_2)_i-C_{3-9}$ cycloalkyle, $-C_{1-6}$ alkyle -OH, $-(CH_2)_i-C_{1-6}$ alkyloxy, $-(CH_2)_i-C_{1-6}$ fluoroalkyle, - $(CH_2)_i-O-C_{1-6}$ fluoroalkyle, $COR_a$, CN, $NR_{10}R_{11}$, ou un halogène ou un groupe phenyle ou heteroaryle contenant soit a) de 1 à 4 atomes d'azote soit b) un atome d'oxygène ou de soufre et 1 ou 2 atomes d'azote, les groupes phenyle et heteroaryle pouvant être éventuellement substitués par un à trois groupes $R_c$ identiques ou différents,
- $R_a$ et $R_f$ sont identiques ou différents et représentent un groupe $C_{1-6}$ alkyle, $C_{1-6}$ alkyloxy ou $NR_{12}R_{13}$,
- $R_c$ représente un halogène, un groupe $C_{1-6}$ alkyle, $C_{3-7}$ cycloalkyle, $C_{1-6}$ alkyloxy, - $S(O)_k-C_{1-6}$ alkyle, $C_{1-6}$ fluoroalkyle, $C_{1-6}$ fluoroalkyloxy, $-(CH_2)_g-C_{3-7}$ cycloalkyle, OH, - $(CH_2)_g-C_{3-7}$ cycloalkyle, $C_{1-6}$ alkyle- OH, $-(CH_2)_g$- $C_{1-6}$ alkyloxy, $-(CH_2)_g-C_{1-6}$ fluoroalkyle, $-(CH_2)_w-O-C_{1-6}$ fluoroalkyle, $COR_f$, CN, ou $NR_{14}R_{15}$,
- $R_{10}$, $R_{11}$, $R_{12}$, $R_{13}$, $R_{14}$ et $R_{15}$ sont identiques ou différents et représentent un atome d'hydrogène, un groupe $C_{1-6}$ alkyle, $C_{3-7}$ cycloalkyle, $-(CH_2)_h$- $C_{3-7}$ cycloalkyle ou - $(CH_2)_h-C_{1-6}$ fluoroalkyle,

éventuellement les groupes $R_{10}$ et $R_{11}$ peuvent former avec l'atome d'azote qui les porte un hétérocycle tel que : azetidine, pyrolidine, pipéridine, azepane, morpholine ou pipérazine, éventuellement les groupes $R_{12}$ et $R_{13}$ peuvent former avec l'atome d'azote qui les porte un hétérocycle, tel que : azetidine, pyrolidine, pipéridine, azepane, morpholine ou pipérazine, éventuellement les groupes $R_{14}$ et $R_{15}$ peuvent former avec l'atome d'azote qui les porte un hétérocycle, tel que : azetidine, pyrolidine, pipéridine, azepane, morpholine ou pipérazine,

- g, h, et i sont différents ou identiques et sont égales à 1, 2 ou 3,
- k et 1 sont différents ou identiques et sont égales à 0, 1 ou 2,
- w est égale à 2, 3 ou 4,

ainsi que leurs sels pharmaceutiquement acceptables, solvates ou hydrates et leurs conformères ou rotamères.

**2.** Composés selon la revendication 1 **caractérisés en ce que** le groupe $R_1$ représente un halogène, un groupe methoxy, ethoxy, thiométhyle, thioéthyle ou trifluorométhyle.

**3.** Composés selon la revendication 1 choisis parmi les composés ci-dessous, leurs sels pharmaceutiquement acceptables, solvates, hydrates, conformères et rotamères :

2-[(5-Bromo-pyridin-3-ylamino)-methyl]-phenol
2-[(5-Bromo-pyridin-3-ylamino)-methyl]-3-fluoro-phenol
2-[(5-Bromo-pyridin-3-ylamino)-methyl]-4-fluoro-phenol
2-[(5-Methyl-pyridin-3-ylamino)-methyl]-phenol
2-[(5-Bromo-pyridin-3-ylamino)-methyl]-5-fluoro-phenol
2-[(5-Bromo-pyridin-3-ylamino)-methyl]-3,5-dichloro-phenol
2-[(5-Bromo-pyridin-3-ylamino)-methyl]-4-chloro-phenol
2-[(5-Bromo-pyridin-3-ylamino)-methyl]-4,6-difluoro-phenol
2-[1-(5-Bromo-pyridin-3-ylamino)-propyl]-phenol
2-[1-(5-Bromo-pyridin-3-ylamino)-ethyl]-4-fluoro-phenol
2-[(5-Methoxy-pyridin-3-ylamino)-methyl]-phenol
2-[1-(5-Bromo-pyridin-3-ylamino)-ethyl]-phenol
2-[(5-Bromo-pyridin-3-ylamino)-methyl]-3,4-difluoro-phenol
5-(2-Hydroxy-benzylamino)-nicotinonitrile
2-[(5-Chloro-pyridin-3-ylamino)-methyl]-phenol.

**4.** Composés selon l'une des revendications précédentes, en tant que médicaments.

**5.** Utilisation cosmétique d'un composé tel que défini selon l'une des revendications 1 à 3 pour l'hygiène corporelle ou capillaire.

**6.** Utilisation d'un composé selon l'une quelconque des revendications 1 à 3 pour la fabrication d'un médicament pour prévenir et/ou traiter l'hirsutisme, l'alopécie androgénique, de l'hyperpilosité, la dermatite atopique, les désordres de la glande sébacée tels que l'hyperséborrhée, l'acné, la peau grasse ou la dermite séborrhéique.

**7.** Utilisation d'un composé selon l'une quelconque des revendications 1 à 3 pour la fabrication d'un médicament pour traiter l'acné.

**8.** Composé selon l'une quelconque des revendications 1 à 3 pour une utilisation dans la prévention et/ou le traitement de l'hirsutisme, l'alopécie androgénique, de l'hyperpilosité, la dermatite atopique, les désordres de la glande sébacée tels que l'hyperséborrhée, l'acné, la peau grasse ou la dermite séborrhéique.

**9.** Composé selon l'une quelconque des revendications 1 à 3 pour une utilisation dans le traitement de l'acné.

## Patentansprüche

**1.** Verbindungen der Formel (I):

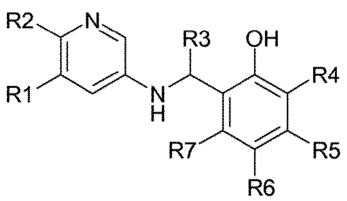

(I)

in der:

- $R_1$ für ein Halogen, eine Methyl-, Ethyl-, Isopropyl-, Trifluormethyl-, Nitril-, Nitro-, Methoxy-, Ethoxy-, Isopropoxy-, Thiomethyl-, Thioethyl- oder Thioisopropyl-Gruppe steht;

- $R_2$ für ein Wasserstoffatom steht;
- $R_3$ für ein Wasserstoffatom oder eine $C_{1-6}$-Alkyl-Gruppe steht;
- $R_4$, $R_5$, $R_6$, $R_7$ identisch oder verschieden sind und entweder für ein Wasserstoffatom oder eine $C_{1-6}$-Alkyl-, $C_{3-7}$-Cycloalkyl-, $C_{1-6}$-Alkyloxy-, $-S(O)_1-C_{1-6}$-Alkyl-, $C_{1-6}$-Fluoralkyl-, $C_{1-6}$-Fluoralkyloxy-, $-(CH_2)_i-C_{3-9}$-Cycloalkyl-, $C_{1-6}$-Alkyl-OH, $-(CH_2)_i-C_{1-6}$-Alkyloxy-, - $(CH_2)_i-C_{1-6}$-Fluoralkyl-, $-(CH_2)_i-O-C_{1-6}$-Fluoralkyl-, $COR_a$, CN, $NR_{10}R_{11}$-Gruppe oder ein Halogen oder eine Phenyl- oder Heteroaryl-Gruppe stehen, enthaltend entweder a) 1 bis 4 Stickstoffatome oder b) ein Sauerstoff- oder Schwefelatom und 1 oder 2 Stickstoffatome, wobei die Phenyl- und Heteroaryl-Gruppen eventuell mit ein bis drei identischen oder verschiedenen $R_c$-Gruppen substituiert sein können,
- $R_4$ und $R_1$ identisch oder verschieden sind und für eine $C_{1-6}$-Alkyl-, $C_{1-6}$-Alkyloxy- oder $NR_{12}R_{13}$-Gruppe stehen;
- $R_c$ für ein Halogen, eine $C_{1-6}$-Alkyl-, $C_{3-7}$-Cycloalkyl-, $C_{1-6}$-Alkyloxy-, $-S(O)_k-C_{1-6}$-Alkyl-, $C_{1-6}$-Fluoralkyl-, $C_{1-6}$-Fluoralkyloxy-, $-(CH_2)_g-C_{3-7}$-Cycloalkyl-, OH, $-(CH_2)_g-C_{3-7}$-Cycloalkyl-, $C_{1-6}$-Alkyl-OH, $-(CH_2)_g-C_{1-6}$-Alkyloxy-, $-(CH_2)_g-C_{1-6}$-Fluoralkyl-, $-(CH_2)_w-O-C_{1-6}$-Fluoralkyl-, $COR_f$, CN, oder $NR_{14}R_{15}$-Gruppe steht,
- $R_{10}$, $R_{11}$, $R_{12}$, $R_{13}$, $R_{14}$ und $R_{15}$ identisch oder verschieden sind und für ein Wasserstoffatom, eine $C_{1-6}$-Alkyl-, $C_{3-7}$-Cycloalkyl-, $-(CH_2)_h-C_{3-7}$-Cycloalkyl- oder $-(CH_2)_h-C_{1-6}$-FluoralkylGruppe stehen,
eventuell die Gruppen $R_{10}$ und $R_{11}$ mit dem Stickstoffatom, das sie trägt, einen Heterocyclus wie Azetidin, Pyrrolidin, Piperidin, Azepan, Morpholin oder Piperazin bilden können, eventuell die Gruppen $R_{12}$ und $R_{13}$ mit dem Stickstoffatom, das sie trägt, einen Heterocyclus wie Azetidin, Pyrrolidin, Piperidin, Azepan, Morpholin oder Piperazin bilden können, eventuell die Gruppen $R_{14}$ und $R_{15}$ mit dem Stickstoffatom, das sie trägt, einen Heterocyclus wie Azetidin, Pyrrolidin, Piperidin, Azepan, Morpholin oder Piperazin bilden können,
- g, h und i verschieden oder gleich sind und gleich 1, 2 oder 3 sind,
- k und 1 verschieden oder gleich sind und gleich 0, 1 oder 2 sind,
- w gleich 2, 3 oder 4 ist,

sowie ihre pharmazeutisch verträglichen Salze, Solvate oder Hydrate und ihre Konformere und Rotamere.

**2.** Verbindungen gemäß Anspruch 1, **dadurch gekennzeichnet, dass** die Gruppe $R_1$ für ein Halogen, eine Methoxy-, Ethoxy-, Thiomethyl-, Thioethyl- oder Trifluormethyl-Gruppe steht.

**3.** Verbindungen gemäß Anspruch 1, ausgewählt aus nachstehenden Verbindungen, ihren pharmazeutisch verträglichen Salzen, Solvaten, Hydraten, Konformeren und Rotameren:

2-[(5-Brom-pyridin-3-ylamino)-methyl]-phenol
2-[(5-Brom-pyridin-3-ylamino)-methyl]-3-fluor-phenol
2-[(5-Brom-pyridin-3-ylamino)-methyl]-4-fluor-phenol
2-[(5-Methyl-pyridin-3-ylamino)-methyl]-phenol
2-[(5-Brom-pyridin-3-ylamino)-methyl]-5-fluor-phenol
2-[(5-Brom-pyridin-3-ylamino)-methyl]-3,5-dichlor-phenol
2-[(5-Brom-pyridin-3-ylamino)-methyl]-4-chlor-phenol
2-[(5-Brom-pyridin-3-ylamino)-methyl]-4,6-difluor-phenol
2-[1-(5-Brom-pyridin-3-ylamino)-propyl]-phenol
2-[1-(5-Brom-pyridin-3-ylamino)-ethyl]-4-fluor-phenol
2-[(5-Methoxy-pyridin-3-ylamino)-methyl]-phenol
2-[1-(5-Brom-pyridin-3-ylamino)-ethyl]-phenol
2-[(5-Brom-pyridin-3-ylamino)-methyl]-3,4-difluor-phenol
5-(2-Hydroxy-benzylamino)-nicotinnitril
2-[(5-Chlor-pyridin-3-ylamino)-methyl]-phenol.

**4.** Verbindungen gemäß einem der vorhergehenden Ansprüche als Medikamente.

**5.** Kosmetische Verwendung einer Verbindung, definiert gemäß einem der Ansprüche 1 bis 3, für die Körper- oder Haarpflege.

**6.** Verwendung einer Verbindung gemäß einem der Ansprüche 1 bis 3 für die Herstellung eines Medikaments zur Prävention und/oder Behandlung von Hirsutismus, Alopecia androgenetica, übermäßiger Behaarung, atopischer Dermatitis, Störungen der Talgdrüse wie Hyperseborrhö, Akne, fettiger Haut oder Dermatitis seborrhoica.

**7.** Verwendung einer Verbindung gemäß einem der Ansprüche 1 bis 3 für die Herstellung eines Medikaments zur Behandlung der Akne.

**8.** Verbindung gemäß einem der Ansprüche 1 bis 3 für eine Verwendung in der Prävention und/oder Behandlung von Hirsutismus, Alopecia androgenetica, übermäßiger Behaarung, atopischer Dermatitis, Störungen der Talgdrüse wie Hyperseborrhö, Akne, fettiger Haut oder Dermatitis seborrhoica.

**9.** Verbindung gemäß einem der Ansprüche 1 bis 3 für eine Verwendung in der Behandlung der Akne.

**Claims**

**1.** Compounds of formula (I):

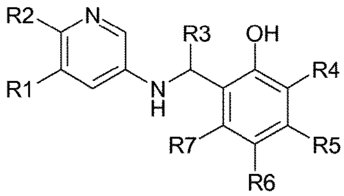

(I)

wherein:

- $R_1$ represents a halogen, a methyl, ethyl, isopropyl, trifluoromethyl, nitrile, nitro, methoxy, ethoxy, iso-propoxy, thiomethyl, thioethyl or thio isopropyl group;
- $R_2$ represents a hydrogen atom;
- $R_3$ represents a hydrogen atom or a $C_{1-6}$ alkyl group;
- $R_4$, $R_5$, $R_6$, $R_7$ are identical or different and represent either a hydrogen atom or a $C_{1-6}$ alkyl, $C_{3-7}$ cycloalkyl, $C_{1-6}$ alkyloxy, $-S(O)_1-C_{1-6}$ alkyl, $C_{1-6}$ fluoroalkyl, $C_{1-6}$ fluoroalkyloxy, $-(CH_2)_i-C_{3-9}$ cycloalkyl, $C_{1-6}$ alkyl-OH, $-(CH_2)_i-_{1-6}$ alkyloxy, $-(CH_2)_i-C_{1-6}$ fluoroalkyl, $-(CH_2)_i-O-C_{1-6}$ fluoroalkyl, $COR_a$, CN, $NR_{10}R_{11}$ group, or halogen or a phenyl or heteroaryl group containing either a) of 1 to 4 carbon nitrogen or b) an oxygen atom or sulfur atom and 1 or 2 nitrogen atoms, these groups phenyl and heteroaryl can optionally be substituted by one to three identical or different groups Rc
- $R_a$ and $R_f$ are identical or different and represent a $C_{1-6}$ alkyl, $C_{1-6}$ alkyloxy or $NR_{12}R_{13}$ group,
- $R_c$ represents halogen, a $C_{1-6}$ alkyl, $C_{3-7}$ cycloalkyl, $C_{1-6}$ alkyloxy, $-S(O)_k-C_{1-6}$ alkyl, $C_{1-6}$ fluoroalkyl, $C_{1-6}$ fluoroalkyloxy, $-(CH_2)_g-C_{3-7}$ cycloalkyl, OH, $-(CH_2)_g-C_{3-7}$ cycloalkyl, $C_{1-6}$ alkyl-OH, $-(CH_2)_g-C_{1-6}$ alkyloxy, $-(CH_2)_g-C_{1-6}$ fluoroalkyl, $-(CH_2)_w-O-C_{1-6}$ fluoroalkyl, $COR_f$, CN, or $NR_{14}R_{15}$ group,
- $R_{10}$, $R_{11}$, $R_{12}$, $R_{13}$, $R_{14}$ and $R_{15}$ are identical or different and represent a hydrogen atom, a $C_{1-6}$ alkyl, $C_{3-7}$ cycloalkyl, $-(CH_2)_h-C_{3-7}$ cycloalkyl or $-(CH_2)_h-C_{1-6}$ fluoroalkyl group,
optionally the groups $R_{10}$ and $R_{11}$ may form with the nitrogen atom which carries them a heterocycle as: azetidine, pyrrolidine, piperidine, azepane, morpholine or piperazine, optionally the groups $R_{12}$ and $R_{13}$ may form with the nitrogen atom which carries them a heterocycle as: azetidine, pyrrolidine, piperidine, azepane, morpholine or piperazine, optionally the groups $R_{14}$ and $R_{15}$ may form, with the nitrogen atom which carries them a heterocycle as: azetidine, pyrrolidine, piperidine, azepane, morpholine or piperazine,
- g, h, and i are identical or different and are 1, 2 or 3
- k and l are identical or different and are 0, 1 or 2
- w is equal to 2, 3 or 4

and their pharmaceutically acceptable salts, hydrates or solvates and their conformers or rotamers.

**2.** Compounds according to claim 1 **characterized in that** the group $R_1$ represents halogen, a methoxy, ethoxy, thiomethyl, thioethyl or a trifluoromethyl.

**3.** Compounds according to claim 1 selected from the following compounds, their pharmaceutically acceptable salts, solvates, hydrates, conformers and rotamers:

2-[(5-Bromo-pyridin-3-ylamino)-methyl]-phenol

2-[(5-Bromo-pyridin-3-ylamino)-methyl]-3-fluoro-phenol
2-[(5-Bromo-pyridin-3-ylamino)-methyl]-4-fluoro-phenol
2-[(5-Methyl-pyridin-3-ylamino)-methyl]-phenol
2-[(5-Bromo-pyridin-3-ylamino)-methyl]-5-fluoro-phenol
2-[(5-Bromo-pyridin-3-ylamino)-methyl]-3,5-dichloro-phenol
2-[(5-Bromo-pyridin-3-ylamino)-methyl]-4-chloro-phenol
2-[(5-Bromo-pyridin-3-ylamino)-methyl] -4,6-difluoro-phenol
2-[1-(5-Bromo-pyridin-3-ylamino)-propyl]-phenol
2-[1-(5-Bromo-pyridin-3-ylamino)-ethyl]-4-fluoro-phenol
2-[(5-Methoxy-pyridin-3-ylamino)-methyl]-phenol
2-[1-(5-Bromo-pyridin-3-ylamino)-ethyl]-phenol
2-[(5-Bromo-pyridin-3-ylamino)-methyl] -3,4-difluoro-phenol
5-(2-Hydroxy-benzylamino)-nicotinonitrile
2-[(5-Chloro-pyridin-3-ylamino)-methyl]-phenol

**4.** Compounds according to one of the preceding claims, as medicaments.

**5.** Cosmetic use of a compound as defined according to any one of claims 1 to 3 for body or hair hygiene.

**6.** Use of a compound according to any one of claims 1 to 3 for the manufacture of a medicament for preventing and/or treating hirsutism, androgenic alopecia, hyperpilosity, atopic dermatitis, disorders of the Sebaceous gland such as the hyperseborrhoea of acne, oily skin or seborrheic dermatitis.

**7.** Use of a compound according to any one of claims 1 to 3 for the manufacture of a medicament for treating acne.

**8.** Compound according to any one of claims 1 to 3, for use for preventing and/or treating hirsutism, androgenic alopecia, hyperpilosity, atopic dermatitis, disorders of the Sebaceous gland such as the hyperseborrhoea of acne, oily skin or seborrheic dermatitis.

**9.** Compound according to any one of claims 1 to 3, for use for treating acne.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 580459 A
- WO 200542464 A
- WO 2006010637 A
- US 4578390 A


### Littérature non-brevet citée dans la description


- Handbook of Pharmaceutical Salts : Properties, Selection and Use de Stahl et Wermuth. Wiley-VCH, 2002
- *Org. Pro. R. & D.,* 2006, 971-1031
- Protective Groups in Organic Chemistry. Plenum Press, 1973
- Protective Groups in Organic Synthesis. John Wiley et Sons, 1991
- **KOCIENSKI P.J.** Protecting Groups. Georg Thieme Verlag, 1994
- **MONOD J.** *J. Molecular Biology,* 1965, vol. 12 (1), 88-118